(19) Europäisches Patentamt
European Patent Office
Office européen des brevets

(11) **EP 2 052 680 B1**

(12) **FASCICULE DE BREVET EUROPEEN**

(45) Date de publication et mention
de la délivrance du brevet:
**24.08.2011 Bulletin 2011/34**

(51) Int Cl.:
***A61B 5/0452*** *(2006.01)*

(21) Numéro de dépôt: **08290992.0**

(22) Date de dépôt: **22.10.2008**

(54) **Dispositif électrocardiologique d'aide au diagnostic, notamment pour le diagnostic du syndrome de Brugada et de l'ERS**

Elektrokardiologisches Diagnosehilfsgerät, insbesondere zur Diagnose des Brugada-Syndroms und von ERS

Electrocardiology diagnosis assistance device, in particular for diagnosing Brugada syndrome and ERS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **24.10.2007 FR 0707441**

(43) Date de publication de la demande:
**29.04.2009 Bulletin 2009/18**

(73) Titulaire: **Ela Medical**
**92541 Montrouge (FR)**

(72) Inventeurs:
• **Maison-Blanche, Pierre**
**75017 Paris (FR)**
• **Extramiana, Dominique**
**94100 Saint Maur des Fosses (FR)**

(74) Mandataire: **Dupuis-Latour, Dominique et al**
**SEP Bardehle Pagenberg Dost Altenburg Geissler**
**10, boulevard Haussmann**
**75009 Paris (FR)**

(56) Documents cités:
**EP-A- 0 472 411       EP-A- 1 543 770**
**EP-A- 1 752 094       WO-A-2006/081336**

**Description**

L'invention concerne le domaine de l'électrophysiologie.

Elle a pour objet un dispositif d'aide au diagnostic de certains troubles du rythme cardiaque, comportant des moyens de caractérisation de l'onde de repolarisation ventriculaire (onde T) d'un signal ECG recueilli sur un patient.

Le signal ECG peut être recueilli de diverses manières : par des électrodes appliquées sur le corps du patient et reliées à un enregistreur externe, ou bien depuis un dispositif implanté pourvu d'électrodes de recueil d'ECG subcutané, ou encore par reconstruction des signaux ECG à partir de signaux EGM endocavitaires recueillis par une prothèse implantée. Dans tous les cas, le signal ECG est échantillonné et numérisé pour être ensuite analysé automatiquement, ou affiché sur un écran à la disposition du praticien.

Divers dispositifs ont déjà été proposés pour analyser spécifiquement l'onde T, par exemple celui décrit dans le FR 2 784 035 A1 (ELA Medical), afin de détecter l'apparition d'un état ischémique, puis l'évolution de cet état en temps réel pour obtenir rapidement un prédicteur fiable de fibrillation ou d'ischémie chez le patient. Ou encore, le dispositif décrit dans le EP 1 486 166 A1 (ELA Medical), qui cherche à évaluer l'*alternans,* très légère variation répétitive d'un battement au suivant, de l'ordre du millivolt, de la forme d'onde de l'ECG dans le segment temporel correspondant à l'onde de repolarisation ; la présence d'un tel phénomène révèle une repolarisation non uniforme du myocarde et constitue un très bon prédicteur de fibrillation, donc du risque clinique d'arythmie ventriculaire et de mort subite.

Un autre paramètre de l'onde T qu'il peut être intéressant d'analyser est l'élévation du segment ST.

La quantification de ce paramètre est notamment un indice-clef dans le diagnostic de la pathologie dite "syndrome de Brugada". Cette pathologie, d'origine génétique, est caractérisée par des anomalies du segment ST entraînant un risque élevé d'arythmie ventriculaire et de mort subite. Les manifestations en apparaissent généralement durant l'âge adulte, avec un risque élevé, notamment chez les patients d'origine asiatique, de mort subite vers la quarantaine par fibrillation ventriculaire, survenant généralement au cours du sommeil et sans antécédent de syncope ou d'arrêt cardiaque.

Il serait souhaitable de pouvoir quantifier les anomalies du segment ST et, parmi les patients porteurs du syndrome, d'identifier ceux présentant une probabilité élevée de survenue d'arythmies ventriculaires et de mort subite, de manière à pouvoir leur proposer un traitement préventif, notamment par pose d'un défibrillateur implanté.

Il est généralement admis que le syndrome de Brugada est caractérisé par une élévation du segment ST supérieure à 200 µV sur les dérivations précordiales, apparaissant sur au moins deux de ces dérivations (cf. Antzelevitch et coll., Brugada Syndrome: Report of the 2nd Consensus Conference, Circulation 2005; 111:659-670).

Un syndrome comparable, même si les causes profondes et les populations concernées sont différentes, est celui dénommé "Early Repolarization Syndrome" (ERS) ou "syndrome de Haïssaguerre", dont les symptômes (notamment syncopes et élévation du segment ST à la lecture de l'ECG), de même que les complications (notamment la mort subite) sont identiques à ceux du syndrome de Brugada. On pourra notamment se référer à : Haïssaguerre et coll., Sudden Cardiac Arrest Associated with Early Repolarization, New England Journal of Medicine 2008 ; 358:2016-2023. Ces syndromes, qui concernent des populations différentes, sont tous deux d'origine génétique, même si le ou les gènes directement impliqués dans l'ERS n'ont pas été isolés à ce jour.

De nombreuses techniques ont été proposées pour quantifier l'élévation du segment ST, que ce soit à partir des bandes d'enregistrement des ECG sur papier (situation la plus couramment rencontrée en clinique), mais également à partir d'ECG numérisés. Concrètement, la plupart des praticiens interprètent l'ECG en utilisant le quadrillage de la bande d'enregistrement et une règle pour quantifier cette élévation du segment ST chez les patients à risque. Comme on le comprend aisément, une telle interprétation visuelle et manuelle de l'élévation du segment ST est sujette à une très grande variabilité d'un patient à l'autre, et de plus elle requiert un temps d'analyse important et ne permet pas de détecter de variations fines du segment ST.

L'article de Kaneko et coll., Automated Detection of Brugada-Type Electrocardiogram Using Diagnostic Criteria of the European Society of Cardiology and the American Heart Association, Journal of Electrocardiology 38 (2005) 96-99 décrit une technique d'évaluation automatique de l'élévation du segment ST, mais par analyse de l'ECG de repos uniquement, et en se basant sur une référence temporelle entachée d'une incertitude non négligeable, à savoir le point J, c'est-à-dire le point de jonction entre le complexe QRS et le segment ST de l'onde cardiaque.

Une autre technique d'analyse de l'élévation du segment ST est décrite dans le EP 1 752 094 A1, dans le but de caractériser l'insuffisance rénale aigüe par analyse de l'électrogramme recueilli par un implant cardiaque sur la base de divers paramètres incluant (entre autres) l'élévation du segment ST.

Le WO 2006/081336 A2 décrit une technique comparable, dans le but de discriminer un état d'ischémie cardiaque d'une situation d'hypo- ou hyperglycémie, susceptible également d'influer sur l'élévation du segment ST.

De façon générale, l'invention a pour objet un dispositif électrocardiologique de quantification automatique de l'élévation du segment ST sur les dérivations ECG précordiales droites, par une technique qui soit à l'abri de tout biais d'interprétation par le praticien, ceci avec une sensibilité élevée, de l'ordre de 10 µV, et avec mise à disposition immédiate des analyses effectuées sur le segment ST.

Comme on le verra, l'invention propose un dispositif permettant de :

- quantifier de manière fiable des anomalies d'un électrocardiogramme observées sur le segment ST chez des patients présentant un risque de syndrome de Brugada ou d'ERS ;
- utiliser les données de cette quantification pour classer les patients en différentes catégories de risque afin d'aider le praticien dans le choix du traitement à prescrire, notamment le point de savoir s'il y a lieu ou non d'implanter un défibrillateur chez le patient ;
- pondérer les indications fournies par le dispositif en fonction de paramètres secondaires tels que le rythme cardiaque du patient, son niveau d'activité, la période de la journée (veille/sommeil, prise de repas,...);
- produire des affichages graphiques donnant au praticien une vision immédiate de l'évolution et des tendances sur le long terme d'un indice de sévérité de la pathologie ;
- suivre les effets de traitements divers prescrits au patient, par exemple l'administration d'une substance anti-arythmique, sur le degré de gravité de la pathologie, afin d'évaluer la pertinence de cette prescription et en adapter éventuellement la posologie.

Bien que l'invention soit principalement décrite dans une application à l'aide au diagnostic du syndrome de Brugada ou de l'ERS, on notera que cette application n'est en aucune façon limitative, et que l'invention peut être pour d'autres types de diagnostics, dès lors que ces diagnostics impliquent une caractérisation du segment ST d'un signal ECG.

Dans le cas du diagnostic du syndrome de Brugada ou de l'ERS, outre la multiplicité des critères et la nécessité de valider des critères annexes, une première difficulté tient au fait que l'anomalie du segment ST caractéristique de ces deux syndromes ne s'exprime pas de manière permanente. Il est donc nécessaire d'exploiter de longues périodes de signal ECG pour détecter ce type de morphologie particulière de l'onde T.

Une autre difficulté tient au fait que la définition actuellement acceptée de l'anomalie caractéristique du segment ST se base sur une "élévation" de ce segment, donc un paramètre de tension (en ordonnée sur l'enregistrement), sans véritable définition précise de l'origine temporelle (en abscisse sur l'enregistrement) servant de référence aux mesures opérées. En particulier, comme on l'exposera par la suite, le "point J" correspondant à la jonction entre le complexe QRS et le segment ST, est souvent difficile à identifier sur les enregistrements et peut également varier, du point de vue temporel, d'une dérivation à l'autre.

Les techniques décrites par les EP 1 752 094 A1 et WO 2006/081336 A2 précités, qui visent la détection d'autres types de pathologies, ne permettent pas de résoudre cette difficulté.

Pour remédier à cette difficulté, Kaneko et coll. (article précité) proposent de "moyenner" la position du point J sur la base de plusieurs dérivations, mais il s'agit là d'une approximation qui en pratique conduit à des incertitudes importantes et imprévisibles sur les résultats finals obtenus.

En fait, la présente invention vise à opérer un diagnostic de phénomènes de nature transmembranaire, et non des phénomènes liés à des cellules malades comme dans le cas de pathologies telles que l'insuffisance rénale ou l'ischémie cardiaque. En effet, les pathologies liées au syndrome de Brugada ou à l'ERS, qui se manifestent dans des coeurs sains, sont causées par des "channelopathies" ou dysfonctionnements des canaux ioniques.

l'invention n'a pas non plus pour but de caractériser par exemple un état ischémique ou d'insuffisance rénale et suivre son évolution dans le temps, mais d'assurer une prévention et/ou un pronostic de mort subite, chez des patients qui très souvent ne présentent aucun symptôme particulier.

Pour atteindre les buts ci-dessus, l'invention propose un dispositif électrocardiologique d'aide au diagnostic comportant des moyens de caractérisation de l'onde de repolarisation ventriculaire d'un signal ECG recueilli sur un patient et préalablement échantillonné et numérisé, du type général divulgué par le EP 1 752 094 A1 précité, comprenant les éléments indiqués dans le préambule de la revendication 1, à savoir :

- des moyens extracteurs, aptes à isoler du signal ECG, pour chaque battement cardiaque, un segment ST formé d'une succession d'échantillons de l'onde de repolarisation ventriculaire, pris à l'intérieur d'une fenêtre temporelle de durée prédéterminée s'étendant à partir d'un instant de début de fenêtre défini par un décalage appliqué à une origine temporelle prédéterminée ;
- des moyens de quantification, aptes à calculer un indice d'élévation dudit segment par rapport à un niveau de référence prédéterminé ; et
- des moyens d'analyse, aptes à analyser sur une succession de battements cardiaques la persistance et/ou la variation dudit indice d'élévation.

De façon caractéristique de l'invention, l'origine temporelle prédéterminée est l'instant d'apparition du complexe QRS, et le dispositif comprend en outre des moyens pour déterminer la position temporelle de ce point sur ledit signal ECG à chaque battement cardiaque.

Des caractéristiques subsidiaires avantageuses sont énoncées dans les sous-revendications.

Le dispositif incorporant les enseignements de l'invention peut être :

- un enregistreur externe apte à être relié à des électrodes externes de recueil d'ECG cutané ;
- un dispositif implanté pourvu d'électrodes de recueil d'ECG subcutané ;
- un dispositif implanté apte à être relié à des électrodes de recueil de signaux EGM endocavitaires ou épicardiques, et comprenant des moyens de reconstruction de signaux ECG à partir desdits signaux EGM recueillis ;
- un dispositif implanté de type stimulateur et/ou défibrillateur comprenant des moyens de délivrance d'impulsions de stimulation et/ou d'un choc de défibrillation, contrôlés par lesdits moyens de caractérisation de l'onde de repolarisation ventriculaire.

◊

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est une représentation schématique du thorax d'un patient sur lequel ont été appliquées des électrodes de recueil d'ECG reliées à un enregistreur externe.

La Figure 2 est homologue de la Figure 1, dans le cas où les signaux ECG sont recueillis depuis un dispositif implanté tel qu'un stimulateur.

La Figure 3 montre la forme d'onde typique d'un signal ECG chez un patient sain, avec les différents points caractéristiques de ce signal.

La Figure 4 illustre les formes d'onde obtenues sur les diverses dérivations précordiales, pour un patient présentant un syndrome de Brugada ou un ERS.

La Figure 5 montre, sur une même figure, les variations du potentiel d'onde cellulaire, avec simultanément les formes d'ondes correspondantes sur deux dérivations précordiales.

La Figure 6 illustre la manière de définir une fenêtre temporelle d'analyse du segment ST sur le signal ECG d'une dérivation précordiale, avec la matrice de données numériques correspondante formée à partir des échantillons numérisés.

La Figure 7 montre les variations, sur une période de 24 heures, d'un indice d'élévation du segment ST par rapport à un critère prédéterminé.

La Figure 8 montre les variations, au cours du temps, de cet indice d'élévation pour différentes durées de la fenêtre temporelle d'analyse.

◊

On va maintenant décrire un exemple de réalisation du dispositif de l'invention.

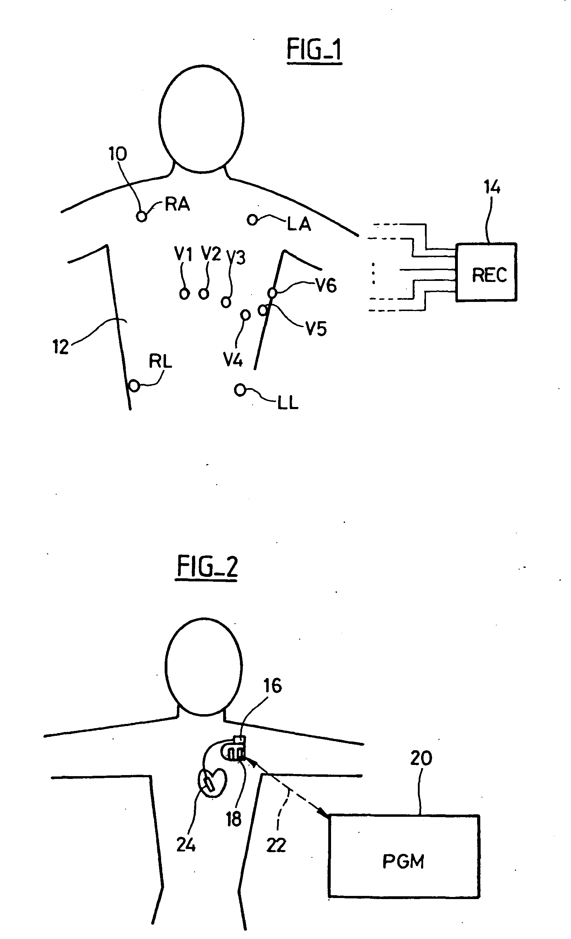

La Figure 1 illustre schématiquement une situation dans laquelle l'invention est mise en oeuvre par recueil d'un signal ECG au moyen d'électrodes cutanées externes appliquées sur le thorax 12 d'un patient.

De façon générale, l'activité électrique cardiaque se manifeste à la surface du corps du patient par des signaux ECG, qui sont recueillis à partir d'électrodes 10 placées en des points particuliers du corps du patient, ces points étant conventionnellement désignés RA, LA, RL, LL, et V1 à V6. Les signaux ECG sont recueillis entre des paires prédéfinies de ces électrodes, chacune de ces paires déterminant une dérivation différente. L'analyse complète de l'ECG implique un ensemble de douze dérivations, de sorte que l'activité électrique cardiaque peut être assimilée à un vecteur en douze dimensions variant au cours du temps : les dérivations bipolaires (I, II, III) et unipolaires (aVF, aVR, aVL) permettent de représenter l'activité électrique dans le plan frontal, tandis que les dérivations précordiales (v1 à v6) la représentent dans le plan horizontal.

Les signaux issus des diverses dérivations sont échantillonnés, numérisés et enregistrés par un dispositif 14, qui peut être un appareil de type électrocardiographe utilisable au cabinet du praticien ou en milieu hospitalier, ou bien un enregistreur ambulatoire de type Holter assurant un enregistrement des données sur une longue durée, typiquement d'une ou plusieurs journées.

On notera que la présente invention n'est pas restreinte à l'analyse d'un ECG standard sur douze dérivations, mais peut également s'appliquer à des ECG produits à partir d'un nombre plus réduit de dérivations. Pour le diagnostic d'une pathologie telle que le syndrome de Brugada ou l'ERS, on peut notamment prendre en compte le fait que les dérivations pertinentes sont les dérivations précordiales v1 à v6, notamment les dérivations standard v1 à v3 qui décrivent l'activité électrique du ventricule droit. Le syndrome de Brugada et l'ERS sont en effet des pathologies liées à un dysfonctionnement du courant ionique transmembranaire qui se manifeste tout particulièrement par des modifications sur l'ECG ventriculaire droit.

La Figure 2 est homologue de la Figure 1, dans le cas où les signaux ECG sont recueillis par un dispositif médical implantable actif 16, typiquement un stimulateur ou défibrillateur cardiaque implantable.

La prothèse 16 peut notamment être pourvue sur son boîtier d'électrodes 18 de recueil d'un ECG subcutané.

L'ECG est alors obtenu directement depuis l'intérieur du corps du patient au lieu de l'être, comme dans le cas de la Figure 1, par des électrodes de surface appliquées sur la peau. L'ECG ainsi recueilli est exploité et/ou mémorisé dans la prothèse implantable 16, pour être ultérieurement lu par un dispositif externe ou programmateur 20 couplé à la prothèse 16 par une liaison non galvanique 22, telle qu'une liaison inductive ou une liaison radiofréquence.

En variante, et notamment pour les prothèses ne comportant pas d'électrodes de recueil d'ECG subcutané, il est possible de recueillir des signaux d'électrogramme endocavitaire ou épicardique (EGM), qui sont des signaux recueillis par des électrodes endocavitaires ou épicardiques 24. Ces signaux, directement issus de l'activité électrique des cellules cardiaques, sont typiquement utilisés pour piloter la prothèse et pour diagnostiquer certains troubles du rythme nécessitant le déclenchement automatique d'une thérapie antitachycardique, antibradycardique ou de resynchronisation interventriculaire. Cependant les signaux ECG et EGM, bien qu'ils aient la même source (l'activité électrique du myocarde), se présentent visuellement de façon assez différente. Mais l'on a développé des algorithmes de reconstruction de l'ECG de surface à partir des signaux EGM, comme décrit par exemple dans les US 5 740 811 B1 (Hedberg) et US 6 980 850 B1 (Kroll), qui proposent des moyens pour émuler ou synthétiser un ECG de surface à partir des signaux EGM recueillis par une prothèse implantée. On peut également faire référence au EP 1 902 750 A1 (demande déposée le 11.09.2007 et publiée le 26.03.2008), qui décrit encore une autre technique de reconstruction d'un ECG à partir d'un EGM, permettant de pallier la plupart des déficiences et limitations des algorithmes proposés jusqu'à présent.

Les signaux ECG reconstitués pourront être utilisés dans le cadre de l'invention de la même manière que ceux délivrés directement à partir d'électrodes cutanées externes.

Il est en outre possible d'utiliser une paire d'électrodes dédiées, fournissant l'information la plus pertinente et permettant à elles seules de délivrer les informations utiles des dérivations v1 et v2, ou de reconstruire le signal d'une telle dérivation sur la base d'un EGM par un algorithme de reconstruction approprié.

Quel que soit le mode de recueil (électrodes externes, électrodes subcutanées ou ECG synthétisé à partir d'un EGM), les signaux sont soumis à un traitement préalable d'échantillonnage, de numérisation et de filtrage. Le filtrage peut notamment inclure, de manière en elle-même connue, un filtrage de la composante continue (ligne isoélectrique) et un filtrage passe-bas pour éliminer les bruits de fréquence élevée.

Les données ECG peuvent également être filtrées de manière à éliminer les battements cardiaques d'origine non sinusale (battements ectopiques ventriculaires ou auriculaires). Cette opération peut être obtenue par analyse de la morphologie des complexes cardiaques au moyen d'algorithmes en eux-mêmes connus, de type "scanner Holter".

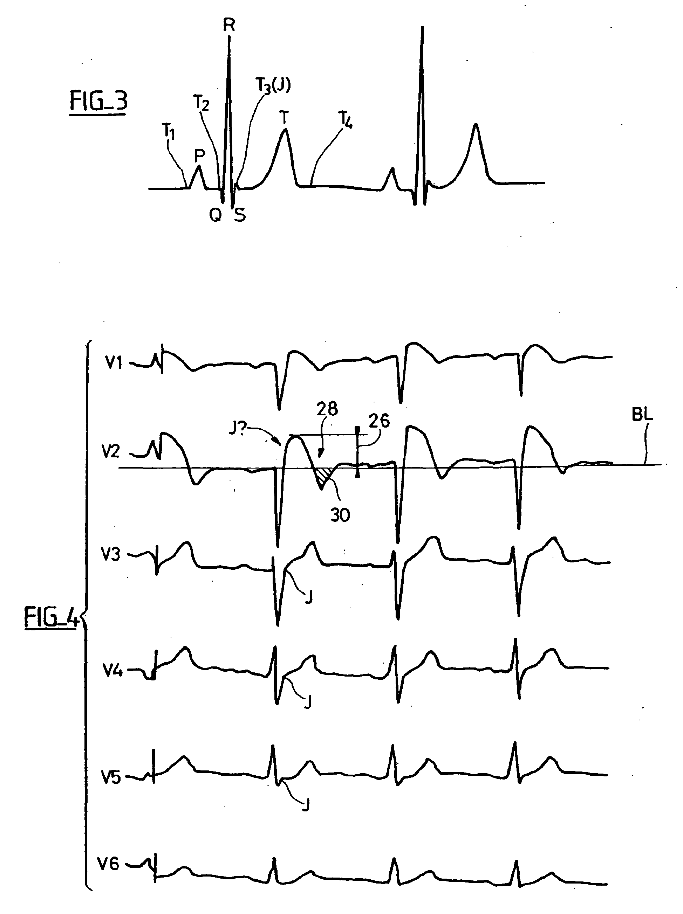

La Figure 3 illustre la morphologie d'un battement cardiaque d'origine sinusale chez un sujet sain.

La contraction auriculaire (onde P) est suivie de la contraction ventriculaire (complexe QRS) puis d'une onde de repolarisation (onde T), qui chez un sujet sain est en forme de cloche (*bell-shaped*). Les différents segments corporels de l'onde sont définis entre les instants $T_1$ (début de l'onde P, *P onset*), $T_2$ (début du complexe QRS, *QRS onset*), $T_3$ ou "point J" (fin du complexe QRS, *QRS offset*) et $T_4$ (fin de l'onde T, *T offset*).

L'invention concerne plus particulièrement l'analyse du segment ST, c'est-à-dire celui défini par l'intervalle [$T_3$, $T_4$].

La position temporelle (en abscisse) des différents points de ce segment ST seront désignés en fonction du nombre de millisecondes suivant le point J (instant $T_3$) : par exemple "J + 60" signifie un point de l'onde situé 60 ms après le point J.

Concrètement, le début du complexe QRS (c'est-à-dire l'instant $T_2$) est en général aisément identifiable sur toutes les dérivations, tandis que la fin du complexe QRS, à l'endroit de la jonction avec le segment ST, est souvent difficile à caractériser de manière précise, en particulier sur les dérivations précordiales.

La Figure 4 montre un exemple de signaux obtenus sur les diverses dérivations précordiales v1 à v6, chez un sujet présentant une pathologie de type syndrome de Brugada ou ERS.

Sur cet exemple, les anomalies caractéristiques de l'onde T sont particulièrement apparentes sur deux des dérivations précordiales (v1 et v2), avec (i) élévation importante (comme en 26) du segment ST par rapport à la ligne isoélectrique ou *baseline* BL, (ii) présence caractéristique d'un co*ved pattern* (forme en dôme) sur l'onde T (comme en 28), et (iii) creusement suivi par une onde T négative (comme en 30).

On notera que sur les différentes dérivations de l'exemple illustré Figure 4, la jonction entre le complexe QRS et le segment ST, c'est-à-dire la point J, est bien apparente sur les dérivations v3 à v5 - permettant donc d'y caractériser le point J dans le temps -, mais pas sur les dérivations v1 et v2 qui, précisément, présentent l'anomalie de l'onde T révélatrice du syndrome que l'on souhaite diagnostiquer.

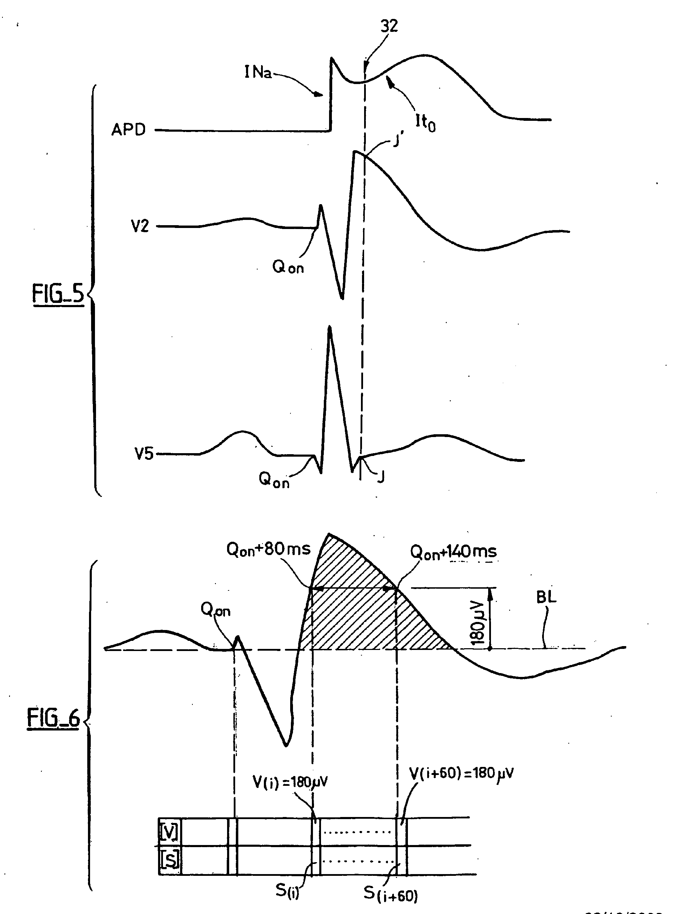

La Figure 5 illustre les variations du potentiel d'onde cellulaire APD (*Action Potential Duration*), avec simultanément les formes d'ondes correspondantes sur deux dérivations précordiales v2 et v5. La comparaison des formes de ces deux dérivations met en lumière le phénomène dit d'*interlead variability,* variabilité d'une forme d'onde à l'autre. Ainsi, le point J déterminé sur la dérivation v5, où ce point est bien apparent et caractérisé, donne sur la dérivation v2 un point J' décalé dans le temps. De ce fait, lorsque l'analyse ne porte que sur des dérivations précordiales droites, l'estimation précise de la position temporelle du point J peut se révéler difficile.

Le mécanisme sous-jacent à ce phénomène et à l'élévation du segment ST a fait l'objet de différentes hypothèses. Selon une première hypothèse, dite de "de la repolarisation" l'amplification du potentiel d'action (*APnotch*) dans le ventricule droit serait liée à un courant vers l'extérieur *Ito* prédominant, non compensé par un courant vers l'intérieur, déficient, des ions sodium *INa* ou calcium *ICa,* ces mécanismes ne survenant que dans les toutes premières phases du potentiel d'action. Selon une autre hypothèse, dite "de la conduction", l'élévation du segment ST serait une conséquence d'un retard de conduction due à une déficience fonctionnelle du canal sodium conduisant à une longue persistance de la dépolarisation dans le ventricule droit après la fin de la dépolarisation du ventricule gauche.

En tout état de cause, ces deux hypothèses soulignent la difficulté qu'il y a à définir le point J de façon précise chez des sujets présentant un syndrome de Brugada ou un ERS, à partir de constatations expérimentales.

Un autre aspect est que le phénomène d'élévation du segment ST est un phénomène relativement étalé dans le temps, qui ne peut être évalué à partir d'un seul échantillon de signal : en d'autres termes, il n'est pas possible d'obtenir un diagnostic fiable sur la base de la mesure du niveau de tension d'un seul point du segment, compte tenu de la trop grande variabilité du phénomène.

Afin de remédier à cette difficulté, et comme cela est illustré en référence à la Figure 6, l'invention propose de définir une fenêtre temporelle de durée prédéterminée pour l'analyse du segment ST.

Toujours selon l'invention, le positionnement temporel de cette fenêtre d'analyse est avantageusement défini non pas par rapport au point J (dont la position est non seulement difficile à identifier, mais varie d'une dérivation à l'autre, comme on l'a expliqué plus haut), mais par rapport au point $Q_{on}$ d'apparition du complexe QRS (point $T_2$ sur la Figure 3).

Ce point est en effet déterminable de façon précise : l'instant $Q_{on}$ d'apparition du complexe QRS est défini comme étant l'instant le plus précoce de variation rapide de tension par rapport à la ligne isoélectrique BL, déterminée à partir des variations du module $V_m$ du vecteur défini à partir des diverses dérivations (ou d'une partie d'entre elles, par exemple les trois dérivations précordiales v1, v2 et v3). Ce module est calculé comme suit :

$$V_m = [ (Dérivation1)^2 + (Dérivation2)^2 + \ldots + (Dérivation12)^2 ]^{1/2}$$

On peut alors définir une fenêtre d'analyse commençant, par exemple, à $t = Q_{ON}+80$ et finissant à $t = Q_{ON}+140$, soit une durée de 60 ms.

Ces valeurs ne sont bien entendu données qu'à titre d'exemple typique, et peuvent être adaptées ou paramétrées en fonction des cas particuliers ou des pathologies spécifiques que l'on souhaite diagnostiquer. On notera en particulier que la durée moyenne d'un QRS chez des patients ne présentant pas de pathologie cardiaque structurelle (comme cela est le cas pour les patients présentant un syndrome de Brugada ou un ERS) est de l'ordre de 100 ms, et que cette durée varie relativement peu avec la fréquence cardiaque. Elle est surtout fonction de l'âge du patient et peut donc être paramétrée une fois pour toutes pour un patient donné.

La définition d'une fenêtre d'analyse telle qu'exposée ci-dessus permet de quantifier les anomalies de l'ECG dans une zone allant, essentiellement, de la fin de la dépolarisation au début de la repolarisation dans le ventricule droit.

Comme illustré en bas de la Figure 6, la fenêtre d'analyse est associée à une matrice d'échantillons numérisés. Cette matrice correspond en fait à une table de deux colonnes et *n* lignes, avec pour chaque ligne *i* le rang $S_i$ de l'échantillon et la valeur $V_i$, en microvolts, de la tension relevée au point correspondant au-dessus de la ligne isoélectrique BL.

Le nombre d'échantillons de cette matrice est directement lié à la longueur de la fenêtre et au taux d'échantillonnage de l'ECG. Pour un taux d'échantillonnage de 1000 Hz (1 échantillon/ms) et pour une longueur de fenêtre d'analyse de 60 ms, la matrice comportera deux colonnes de 61 lignes. En variante, il est possible de sélectionner un sous-ensemble d'échantillons pour réduire le nombre de variables, par exemple un échantillon sur dix, c'est-à-dire que l'on prend les échantillons correspondant aux tensions relevées à $Q_{ON} + 80$, $Q_{ON} + 90$, $Q_{ON} + 100$, .... Inversement, avec un taux d'échantillonnage faible, on pourra opérer un suréchantillonnage, de manière à restituer une matrice de même taille d'un dispositif à l'autre.

Cette matrice peut être soumise à divers traitements et calculs, en fonction du diagnostic recherché.

La matrice peut tout d'abord être transformée par application de diverses fonctions mathématiques, par exemple une analyse en composantes principales (PCA) ou une transformation de Karhunen-Loewe (KLT), ou encore un calcul de dérivée (une dérivée permet en effet d'analyser la vitesse de descente de la pente du segment ST). Les dérivations précordiales droites fournissent par ailleurs un signal tridimensionnel v1 v2 v3 qui peut être traité par une décomposition en valeurs singulières pour obtenir un jeu de vecteurs orthogonaux indépendants, le vecteur présentant la valeur propre la plus élevée pouvant être utilisé pour calculer une matrice à base d'analyse PCA..

Outre le paramètre de tension (élévation du segment ST au-dessus de la ligne isoélectrique BL) il est possible d'analyser des données d'aire correspondant à un produit tension x temps (surface hachurée sur la forme d'onde de la Figure 6).

On sait en effet qu'une telle variable peut être utiles pour la détection d'une hypertrophie ventriculaire gauche et pour la quantification de la repolarisation ventriculaire dans la forme congénitale du syndrome QT long. Dans le cas présent, l'aire sera donnée par le produit des différentes tensions (amplitudes en microvolts) par la durée de la fenêtre d'analyse en millisecondes, l'unité résultante étant une valeur en mV x ms. Ce calcul permet de transformer la matrice en un paramètre unique constituant un indice quantifié représentatif de l'élévation du segment ST pour la forme d'onde considérée.

Cet indice est particulièrement pertinent pour l'aide au diagnostic du syndrome de Brugada ou de l'ERS. En effet, l'activité électrique de la paroi ventriculaire droite chez les patients atteints d'un tel syndrome est un phénomène complexe, et des études expérimentales ont montré la présence d'une hétérogénéité électrique entre l'endocarde, l'épicarde et entre les différents sites. L'intégration du signal ECG par un produit temps x tension reflète mieux les déficiences cellulaires qu'une valeur de tension isolée en un point temporel prédéfini.

Il est possible de caractériser d'autres types de morphologies, par exemple liées à l'amplitude (maximum, minimum), ou de nature temporelle (présence d'un phénomène précoce ou tardif à l'intérieur de la fenêtre d'analyse) dans la mesure où le processus de repolarisation peut varier selon l'échelle temporelle considérée.

Il est également possible, par analyse des variations de la tension à l'intérieur de la fenêtre d'analyse, de rechercher dans le segment ST la présence d'un *coved pattern* (forme en dôme) caractéristique d'un syndrome de Brugada ou d'un ERS, par exemple en recherchant si : $V(Q_{on}+80) > V(Q_{on}+90) > ... > V(Q_{on}+140)$. Ou encore en recherchant si : $dV/dt [Q_{on}+80 ; Q_{on}+140] < 0$ et $|dV/dt [Q_{on}+80 ; Q_{on}+140]| >$ seuil prédéterminé, le tout suivi d'une onde négative (V < 0 dans la région 30 de la figure 4).

Ces différents traitements et calculs permettent, pour chaque battement cardiaque, de calculer un indice d'élévation du segment ST par rapport à une référence prédéterminée.

Pour le diagnostic, il conviendra ensuite d'analyser la persistance et/ou la variation de cet indice sur une succession de battements cardiaques. En effet, comme on l'a indiqué plus haut, dans le cas d'un syndrome de Brugada ou d'un ERS le segment ST présente une variabilité importante d'un battement à l'autre.

Avantageusement, cette analyse des variations à moyen/long terme de l'indice est précédée d'une classification des valeurs d'indice obtenues pour chaque battement.

La distribution des valeurs d'indice relevées peut être notamment opérée entre différentes classes prédéfinies en fonction d'un paramètre donné, par exemple la fréquence cardiaque (plus particulièrement en situation de bradycardie). Dans ce cas, on regroupera par exemple toutes les valeurs d'indice obtenues lorsque la fréquence est à 100 bpm, toutes celles obtenues lorsque la fréquence est à 75 bpm, toutes celles obtenues lorsque la fréquence est à 50 bpm, etc.

D'autres paramètres que la fréquence cardiaque peuvent être utilisés pour définir les différentes classes, notamment :

- l'intervalle RR,
- le type de période (journée/nuit/repas, ...),
- le niveau d'activité du patient,
- sa température corporelle (si des capteurs de mesure de température sont prévus),
- la nature stimulée ou spontanée du battement cardiaque,
- son caractère sinusal ou extrasystolique,
- l'existence d'un traitement médicamenteux (par exemple la quinidine, dont la prescription peut se supplanter à celle de l'implantation d'un stimulateur),
- ou encore des combinaisons de ces divers paramètres, par exemple des classes définies par le produit fréquence x période, etc.

L'analyse de la persistance ou de la variation de l'indice d'élévation du segment ST est alors opérée séparément pour chacune des classes. Ceci permet d'isoler éventuellement des conditions particulières d'apparition du syndrome de Brugada ou de l'ERS, avec une quantification permettant d'évaluer un risque plus ou moins élevé de survenue d'un épisode pathologique.

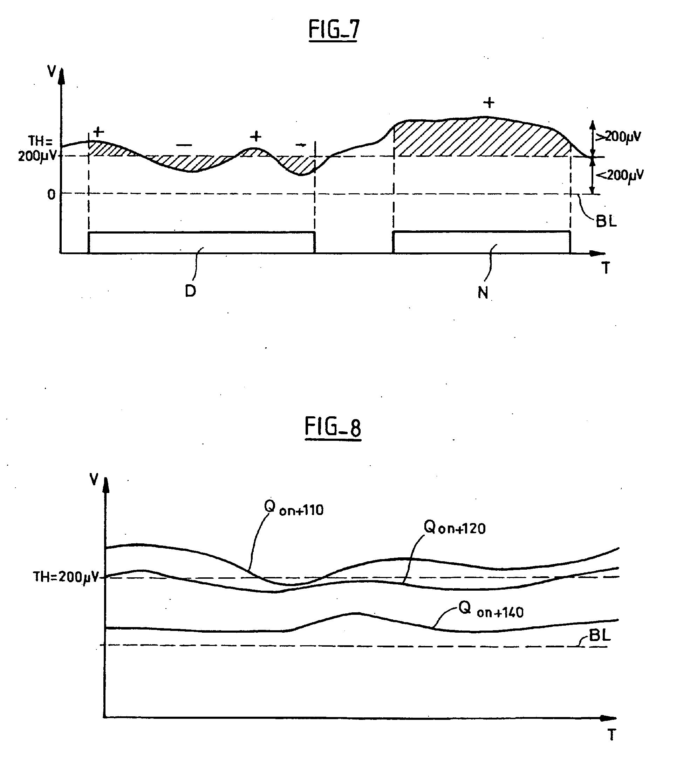

Comme illustré Figure 7, les variations au cours du temps de l'indice d'élévation du segment ST sur une longue durée (typiquement 24 heures au moins) peuvent fournir des informations sur l'évolution d'un risque d'épisode grave, risque reflété par un paramètre désigné "charge de Brugada".

On peut voir notamment sur l'exemple de la figure 7 que dans les périodes diurnes (D) l'élévation du segment ST au-dessus de la ligne isoélectrique BL varie typiquement autour d'un seuil critique TH de 200 μV, mais que dans les périodes nocturnes (N) cette élévation augmente notablement au-dessus de ce même seuil. Les changements de signe du paramètre au-dessus et au-dessous du seuil TH permettent ainsi de détecter l'apparition d'épisodes de Brugada sévères (typiquement, en période nocturne), de manière à prendre toute action préventive appropriée.

Sur la Figure 8, on a représenté les variations au cours du temps des charges de Brugada obtenues en faisant varier la durée de la fenêtre d'analyse, avec une même borne de début mais une borne de fin fixée à $Q_{on}$ + 110, $Q_{on}$ + 120 et $Q_{on}$ + 140 ms. Les différentes valeurs sont présentées séparément, révélant des variations plus ou moins marquées selon le cas. Cette présentation permet notamment au praticien de paramétrer de façon optimale la durée de la fenêtre d'analyse, plus ou moins longue, en fonction de chaque patient soumis au diagnostic.

On va maintenant décrire un essai clinique réalisé en appliquant les enseignements de l'invention, essai qui démontre la pertinence de la caractérisation, de la manière décrite plus haut, de l'élévation du segment ST pour le diagnostic de patients atteints du syndrome de Brugada ou d'un ERS, qu'ils soient symptomatiques ou asymptomatiques.

L'essai a été conduit sur 32 patients de contrôle, sains (16 hommes, âge moyen 50,3 $\pm$ 21,6) et 34 patients atteints du syndrome de Brugada (15 patients symptomatiques et 19 patients asymptomatiques, 30 hommes, âge moyen 46,4 $\pm$ 11,4).

Un enregistrement ECG holter à 12 voies a été réalisé sur l'ensemble de cette population de patients. Les complexes QRST recueillis ont été moyennés chaque minute, donnant 1440 formes d'ondes ECG pour les 24 heures d'enregistrement. Une fenêtre d'analyse a été définie commençant à t = $Q_{on}$ + 80 ms et finissant à t = $Q_{on}$ + 140 ms. Les paramètres suivants ont été relevés : élévation du maximum du segment ST, position temporelle de ce maximum par rapport à $Q_{on}$, pente du segment ST et amplitude du point T.

Les données mesurées sur les voies v1 et v2 sont indiquées dans le TABLEAU 1 ci-dessous (valeur moyenne et écart-type). Par rapport aux patients asymptomatiques, les patients symptomatiques ne montrent pas de tendance significative dans le sens d'une plus grande élévation du maximum du segment ST. Le diagramme QRST des patients symptomatiques est caractérisé par une plus grande prématurité du maximum du segment ST, une plus forte pente descendante du segment ST et une tension plus négative pour la position du point T.

TABLEAU 1

| | | Contrôle N=32 | Asymptomatique N=19 | Symptomatique N=15 |
|---|---|---|---|---|
| v1 | élévation max ST ($\mu$V) | 56$\pm$32 | 202$\pm$121 * | 206$\pm$130 * |
| | position max ST ($Q_{on}$ +xx) | 128$\pm$17 | 109$\pm$13 * | 101$\pm$14 * |
| | pente ($\mu$V/ms) | 0.3$\pm$0.4 | -2.1$\pm$2.0 * | -3.0$\pm$2.7 * |
| | amplitude T ($\mu$V) | 31$\pm$118 | -85$\pm$115 * | -190$\pm$158 * |
| | | | | |
| v2 | élévation max ST ($\mu$V) | 133$\pm$88 | 270$\pm$119 * | 310$\pm$152 * |
| | position max ST ($Q_{on}$ +xx) | 136$\pm$10 | 118$\pm$15 * | 108$\pm$16 * † |
| | pente ($\mu$V/ms) | 1.2$\pm$1.0 | -1.2$\pm$2.4 * | -3.4$\pm$3.0 * † |
| | amplitude T($\mu$V) | 473$\pm$275 | 165$\pm$228 * | -52$\pm$253 * † |
| * : p < 0,05 post-test vs. contrôle | | | | |
| † : p < 0,05 post-test vs. patients asymptomatiques | | | | |

## Revendications

**1.** Un dispositif électrocardiologique d'aide au diagnostic comportant des moyens de caractérisation de l'onde de repolarisation ventriculaire d'un signal ECG recueilli sur un patient, ce signal ECG ayant été préalablement échantillonné et numérisé, ce dispositif comprenant :

- des moyens extracteurs, aptes à isoler du signal ECG, pour chaque battement cardiaque, un segment ST formé d'une succession d'échantillons de l'onde de repolarisation ventriculaire, pris à l'intérieur d'une fenêtre temporelle de durée prédéterminée s'étendant à partir d'un instant de début de fenêtre défini par un décalage appliqué à une origine temporelle prédéterminée ;
- des moyens de quantification, aptes à calculer un indice d'élévation dudit segment par rapport à un niveau de référence prédéterminé ; et
- des moyens d'analyse, aptes à analyser sur une succession de battements cardiaques la persistance et/ou la variation dudit indice d'élévation,

**caractérisé en ce que** :

**EP 2 052 680 B1**

- ladite origine temporelle prédéterminée est l'instant ($Q_{ON}$) d'apparition du complexe QRS ; et
- le dispositif comprend en outre des moyens pour déterminer la position temporelle de ce point sur ledit signal ECG à chaque battement cardiaque.

2. Le dispositif de la revendication 1, où ledit décalage appliqué à l'origine temporelle prédéterminée est compris entre 60 et 100 ms, de préférence 80 ms.

3. Le dispositif de la revendication 1, où ladite durée prédéterminée de la fenêtre temporelle est comprise entre 50 et 80 ms, de préférence 60 ms.

4. Le dispositif de la revendication 1, où les moyens de quantification comprennent des moyens pour former une matrice donnant, pour une série d'échantillons ($S_i$) pris à l'intérieur de ladite fenêtre temporelle, l'amplitude ($V_i$) de l'échantillon correspondant du signal ECG.

5. Le dispositif de la revendication 4, où ladite série d'échantillons est un sous-ensemble des échantillons de ladite succession d'échantillons de l'onde de repolarisation ventriculaire.

6. Le dispositif de la revendication 4, où le dispositif est apte à analyser concurremment une pluralité de signaux ECG issus de différentes dérivations, et comprend des moyens pour appliquer à la matrice une transformation mathématique du groupe comprenant : une analyse en composantes principales, une transformation de Karhunen-Loeve, et un calcul de dérivée.

7. Le dispositif de la revendication 1, où les moyens de quantification comprennent également des moyens intégrateurs aptes à calculer l'aire délimitée par le signal ECG sur la durée de ladite fenêtre temporelle, pour la détermination de l'indice d'élévation.

8. Le dispositif de la revendication 1, où les moyens d'analyse comprennent des moyens pour évaluer un niveau moyen de l'indice d'élévation pendant une durée donnée et/ou sur un nombre de battements cardiaques donné, et pour analyser les variations à long terme de l'indice d'élévation autour de ce niveau moyen.

9. Le dispositif de la revendication 8, où les moyens d'analyse comprennent des moyens classificateurs, aptes à distribuer en une pluralité de classes les valeurs successives de l'indice d'élévation recueillies pendant une durée donnée et/ou sur un nombre de battements cardiaques donné, cette distribution entre classes étant opérée en fonction d'un paramètre mesuré par le dispositif ou connu de celui-ci, et pour analyser la persistance et/ou la variation de l'indice d'élévation séparément pour chaque classe.

10. Le dispositif de la revendication 9, où ledit paramètre est un paramètre du groupe comprenant : la fréquence cardiaque, l'intervalle RR, la nature diurne, nocturne ou prandiale de l'instant d'analyse, le niveau d'activité du patient, sa température corporelle, la nature stimulée ou spontanée du battement cardiaque, le caractère sinusal ou extrasystolique du battement cardiaque, l'existence d'un traitement médicamenteux, et les combinaisons de ces paramètres.

11. Le dispositif de la revendication 1, où les moyens d'analyse comprennent des moyens de suivi des variations de la tension à l'intérieur de la fenêtre d'analyse, aptes à rechercher dans le segment ST la présence d'une forme en dôme caractéristique d'un syndrome de Brugada ou d'un ERS.

12. Le dispositif de la revendication 1, comprenant en outre des moyens d'affichage graphique des variations dans le temps de l'indice d'élévation.

13. Le dispositif de la revendication 1, où les moyens d'analyse sont également aptes à délivrer une information d'aide au diagnostic comprenant un marqueur de risque.

14. Le dispositif de la revendication 13, où l'information comprenant un marqueur de risque est délivrée lorsque l'indice d'élévation calculé par les moyens de quantification dépasse un seuil prédéfini pendant une durée donnée et/ou sur un nombre de battements cardiaques donné.

15. Le dispositif de la revendication 13, où le dispositif est apte à analyser concurremment une pluralité de signaux ECG issus de dérivations précordiales différentes, et où l'information comprenant un marqueur de risque est délivrée

**9**

lorsque l'indice d'élévation calculé par les moyens de quantification dépasse un seuil prédéfini pendant une durée donnée et/ou sur un nombre de battements cardiaques donné, pour des signaux ECG issus d'au moins deux desdites dérivations précordiales.

**16.** Le dispositif de la revendication 1, où le dispositif est un enregistreur externe (14) apte à être relié à des électrodes externes (10) de recueil d'ECG cutané.

**17.** Le dispositif de la revendication 1, où le dispositif est un dispositif implanté (16) pourvu d'électrodes (18) de recueil d'ECG subcutané.

**18.** Le dispositif de la revendication 1, où le dispositif est un dispositif implanté (16) apte à être relié à des électrodes (24) de recueil de signaux EGM endocavitaires ou épicardiques, le dispositif comprenant en outre des moyens de reconstruction de signaux ECG à partir desdits signaux EGM recueillis.

**19.** Le dispositif de la revendication 1, où le dispositif est un dispositif implanté de type stimulateur et/ou défibrillateur, le dispositif comprenant en outre des moyens de délivrance d'impulsions de stimulation et/ou d'un choc de défibrillation, contrôlés par lesdits moyens de caractérisation de l'onde de repolarisation ventriculaire.

**Claims**

**1.** Electrocardiology diagnosis assistance device comprising means for characterizing the ventricular repolarization wave of an ECG signal collected from a patient, this ECG signal having first been sampled and digitized, this device comprising:

- extraction means, capable of isolating from the ECG signal, for each heartbeat, an ST segment formed by a succession of samples of the ventricular repolarisation wave, taken inside a time window of predetermined duration extending from a window start instant defined by an offset applied to a predetermined time origin;
- quantifying means, capable of calculating an elevation index of said segment relative to a predetermined reference level; and
- analysis means, capable of analysing over a succession of heartbeats the persistence and/or the variation of said elevation index,

**characterized in that**:

- said predetermined time origin is the instant ($Q_{ON}$) of appearance of the QRS complex; and
- the device also comprises means for determining the time position of this point on said ECG signal on each heartbeat.

**2.** Device according to Claim 1, in which said offset applied to the predetermined time origin is between 60 and 100 ms, preferably 80 ms.

**3.** Device according to Claim 1, in which said predetermined duration of the time window is between 50 and 80 ms, preferably 60 ms.

**4.** Device according to Claim 1, in which the quantifying means comprise means for forming a matrix which gives, for a series of samples ($S_i$) taken inside said time window, the amplitude ($V_i$) of the corresponding sample of the ECG signal.

**5.** Device according to Claim 4, in which said series of samples is a subset of the samples of said succession of samples of the ventricular repolarisation wave.

**6.** Device according to Claim 4, in which the device is capable of analysing concurrently a plurality of ECG signals from different derivations, and comprises means for applying to the matrix a mathematical transform of the group comprising: a main component analysis, a Karhunen-Loeve transform, and a calculation of drift.

**7.** Device according to Claim 1, in which the quantifying means also comprise integrator means capable of calculating the area delimited by the ECG signal over the duration of said time window, for the determination of the elevation index.

8. Device according to Claim 1, in which the analysis means comprise means for evaluating a mean level of the elevation index during a given duration and/or over a given number of heartbeats, and for analysing the longterm variations of the elevation index around this mean level.

9. Device according to Claim 8, in which the analysis means comprise classifying means, capable of distributing in a plurality of classes the successive values of the elevation index collected during a given duration and/or over a given number of heartbeats, this distribution between classes being based on a parameter measured by the device or a known parameter thereof, and for analysing the persistence and/or the variation of the elevation index separately for each class.

10. Device according to Claim 9, in which said parameter is a parameter from the group comprising:

heart rate, RR interval, diurnal, nocturnal or prandial nature of the analysis instant, the level of activity of the patient, his body temperature, the stimulated or spontaneous nature of the heartbeat, the sinus or extrasystolic nature of the heartbeat, the existence of medicinal treatment, and the combinations of these parameters.

11. Device according to Claim 1, in which the analysis means comprise means for tracking voltage variations inside the analysis window, capable of searching the ST segment for the presence of a dome characteristic of a Brugada syndrome or an ERS.

12. Device according to Claim 1, also comprising means for graphically displaying variations in time of the elevation index.

13. Device according to Claim 1, in which the analysis means are also capable of delivering diagnosis assistance information comprising a risk marker.

14. Device according to Claim 13, in which the information comprising a risk marker is delivered when the elevation index calculated by the quantifying means exceeds a predefined threshold for a given duration and/or over a given number of heartbeats.

15. Device according to Claim 13, in which the device is capable of analysing concurrently a plurality of ECG signals from different precordial derivations, and in which the information comprising a risk marker is delivered when the elevation index calculated by the quantifying means exceeds a predefined threshold for a given duration and/or over a given number of heartbeats, for ECG signals from at least two of said precordial derivations.

16. Device according to Claim 1, in which the device is an external recorder (14) capable of being linked to external electrodes (10) for cutaneous ECG collection.

17. Device according to Claim 1, in which the device is an implanted device (16) provided with electrodes (18) for subcutaneous ECG collection.

18. Device according to Claim 1, in which the device is an implanted device (16) capable of being linked to electrodes (24) for the collection of endocavitary or epicardial EGM signals, the device also comprising means for reconstructing ECG signals from said collected EGM signals.

19. Device according to Claim 1, in which the device is an implanted device of stimulator and/or defibrillator type, the device also comprising means for delivering stimulation pulses and/or a defibrillation shock, controlled by said means for characterizing the ventricular repolarisation wave.

**Patentansprüche**

1. Elektrokardiologische Vorrichtung zur Diagnosehilfe, umfassend Mittel zur Kennzeichnung der ventrikulären Repolarisationswelle eines EKG-Signals, das an einem Patienten aufgenommen wurde, wobei dieses EKG-Signal vorher bemustert und digitalisiert wurde, wobei diese Vorrichtung Folgendes umfasst:

- Extraktionsmittel, die aus dem EKG-Signal für jeden Herzschlag ein Segment ST isolieren können, das von einer Aufeinanderfolge von Mustern der ventrikulären Repolarisationswelle gebildet ist, aufgenommen innerhalb eines Zeitfensters von vorbestimmter Dauer, das sich von einem Anfangsfenstermoment ausgehend erstreckt, der durch einen Versatz definiert ist, der an einem vorbestimmten Zeitanfang angewandt wird;
- Quantifizierungsmittel, die einen Erhöhungsindex des Segments bezogen auf ein vorbestimmtes Referenzniveau berechnen können; und
- Analysemittel, die an einer Aufeinanderfolge von Herzschlägen die Beständigkeit und/oder Veränderung des Erhöhungsindexes analysieren können,

**dadurch gekennzeichnet, dass**:

- der vorbestimmte Zeitanfang der Moment ($Q_{ON}$) des Auftauchens des Komplexes QRS ist; und
- die Vorrichtung ferner Mittel umfasst, um die zeitliche Position dieses Punktes auf dem EKG-Signal bei jedem Herzschlag zu bestimmen.

2. Vorrichtung nach Anspruch 1, bei der der an dem vorbestimmten Zeitanfang angewandte Versatz zwischen 60 und 100 ms, vorzugsweise 80 ms, beträgt.

3. Vorrichtung nach Anspruch 1, bei der die vorbestimmte Dauer des Zeitfensters zwischen 50 und 80 ms, vorzugsweise 60 ms, beträgt.

4. Vorrichtung nach Anspruch 1, bei der die Quantifizierungsmittel Mittel umfassen, um eine Matrix zu bilden, die für eine Reihe von Mustern ($S_i$), die innerhalb des Zeitfensters genommen wurden, die Amplitude ($V_i$) des entsprechenden Musters des EKG-Signals angibt.

5. Vorrichtung nach Anspruch 4, bei der die Musterserie eine Untereinheit der Muster der Aufeinanderfolge von Mustern der ventrikulären Repolarisationswelle ist.

6. Vorrichtung nach Anspruch 4, bei der die Vorrichtung geeignet ist, gleichzeitig eine Vielzahl von EKG-Signalen, die von verschiedenen Ästen kommen, zu analysieren, und Mittel umfasst, um an der Matrix eine mathematische Umformung der Gruppe, umfassend eine Analyse in Hauptkomponenten, eine Karhunen-Loeve-Transformation, und eine Ablenkungsberechnung anzuwenden.

7. Vorrichtung nach Anspruch 1, bei der die Quantifizierungsmittel auch Integrationsmittel umfassen, die geeignet sind, den von dem EKG-Signal begrenzten Bereich über die Dauer des Zeitfensters zu berechnen, um den Erhöhungsindex zu bestimmen.

8. Vorrichtung nach Anspruch 1, bei der die Analysemittel Mittel umfassen, um ein mittleres Niveau des Erhöhungsindexes während einer gegebenen Dauer und/oder über eine gegebene Anzahl von Herzschlägen hinweg zu bewerten und die langfristigen Veränderungen des Erhöhungsindexes um dieses mittlere Niveau zu analysieren.

9. Vorrichtung nach Anspruch 8, bei der die Analysemittel Klassifikationsmittel umfassen, die geeignet sind, die aufeinander folgenden Werte des Erhöhungsindexes, die während einer gegebenen Zeitdauer und/oder über eine gegebene Anzahl von Herzschlägen hinweg gesammelt wurden, in eine Vielzahl von Klassen zu verteilen, wobei diese Verteilung in Klassen in Abhängigkeit von einem Parameter erfolgt, der von der Vorrichtung gemessen wurde oder dieser bekannt ist, und um die Beständigkeit und/oder Veränderung des Erhöhungsindexes getrennt für jede Klasse zu analysieren.

10. Vorrichtung nach Anspruch 9, bei der der Parameter ein Parameter der Gruppe, umfassend die Herzfrequenz, das RR-Intervall, die tägliche, nächtliche oder prandiale Natur des Analysemoments, das Aktivitätsniveau des Patienten, seine Körpertemperatur, die stimulierte oder spontane Natur des Herzschlags, den Sinuscharakter oder extrasystolischen Charakter des Herzschlags, das Bestehen einer medikamentösen Behandlung und die Kombination dieser Parameter, ist.

11. Vorrichtung nach Anspruch 1, bei der die Analysemittel Mittel zur Verfolgung der Veränderungen der Spannung innerhalb des Analysefensters umfassen, die in dem Segment ST das Vorhandensein einer Buckelform suchen können, die für ein Brugada-Syndrom oder ein ERS charakteristisch ist.

12. Vorrichtung nach Anspruch 1, ferner umfassend grafische Anzeigemittel für die zeitlichen Veränderungen des Erhöhungsindexes.

**13.** Vorrichtung nach Anspruch 1, bei der die Analysemittel auch geeignet sind, eine Information zur Diagnosehilfe zu liefern, umfassend einen Risikomarker.

**14.** Vorrichtung nach Anspruch 13, bei der die Information, umfassend einen Risikomarker, geliefert wird, wenn der von den Quantifizierungsmitteln berechnete Erhöhungsindex während einer gegebenen Zeitdauer und/oder über eine gegebene Anzahl von Herzschlägen hinweg eine vordefinierte Schwelle überschreitet.

**15.** Vorrichtung nach Anspruch 13, bei der die Vorrichtung geeignet ist, gleichzeitig eine Vielzahl von EKG-Signalen, die von verschiedenen präkordialen Ästen kommen, zu analysieren, und bei der die Information, umfassend einen Risikomarker, geliefert wird, wenn der von den Quantifizierungsmitteln berechnete Erhöhungsindex während einer gegebenen Zeitdauer und/oder über eine gegebene Anzahl von Herzschlägen hinweg eine für EKG-Signale, die von mindestens zwei der präkordialen Äste kommen, vordefinierte Schwelle überschreitet.

**16.** Vorrichtung nach Anspruch 1, bei der die Vorrichtung ein externes Aufzeichnungsgerät (14) ist, das an externe EKG-Aufnahmeelektroden (10) auf der Haut angeschlossen werden kann.

**17.** Vorrichtung nach Anspruch 1, bei der die Vorrichtung eine implantierte Vorrichtung (16) ist, die mit subkutanen EKG-Aufnahmeelektroden (18) versehen ist.

**18.** Vorrichtung nach Anspruch 1, bei der die Vorrichtung eine implantierte Vorrichtung (16) ist, die geeignet ist, an Elektroden (24) zur Aufnahme von endokavitären oder epikardialen EGM-Signalen angeschlossen zu werden, wobei die Vorrichtung ferner Mittel zur Rekonstruktion von EKG-Signalen aus den aufgenommenen EGM-Signalen umfasst.

**19.** Vorrichtung nach Anspruch 1, bei der die Vorrichtung eine implantierte Vorrichtung des Typs Stimulator und/oder Defibrillator ist, wobei die Vorrichtung ferner Mittel zur Lieferung von Stimulationsimpulsen und/oder eines Defibrillationsstoßes umfasst, die von den Mitteln zur Kennzeichnung der ventrikulären Repolarisationswelle kontrolliert werden.

## FIG_1

## FIG_2

FIG_3

FIG_4

FIG_5

INa

32

APD

Ito

J'

V2

Qon

V5

Qon

J

FIG_6

Qon+80ms

Qon+140ms

BL

Qon

180μV

V(i)=180μV

V(i+60)=180μV

V

S

S(i)

S(i+60)

## FIG_7

## FIG_8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2784035 A1
- EP 1486166 A1
- EP 1752094 A1
- WO 2006081336 A2
- US 5740811 B1, Hedberg
- US 6980850 B1, Kroll
- EP 1902750 A1

**Littérature non-brevet citée dans la description**

- **ANTZELEVITCH.** *Brugada Syndrome: Report of the 2nd Consensus Conference, Circulation,* 2005, vol. 111, 659-670
- **HAÏSSAGUERRE.** Sudden Cardiac Arrest Associated with Early Repolarization. *New England Journal of Medicine,* 2008, vol. 358, 2016-2023
- **KANEKO.** Automated Detection of Brugada-Type Electrocardiogram Using Diagnostic Criteria of the European Society of Cardiology and the American Heart Association. *Journal of Electrocardiology,* 2005, vol. 38, 96-99